Europäisches Patentamt

European Patent Office

Office européen des brevets

(11) Publication number: **0 263 761**
A2

# (12) EUROPEAN PATENT APPLICATION

(21) Application number: 87402232.0

(22) Date of filing: 07.10.87

(51) Int. Cl.⁴: **G 01 N 33/576**
C 07 K 15/04

(30) Priority: 07.10.86 JP 238688/86

(43) Date of publication of application:
13.04.88 Bulletin 88/15

(84) Designated Contracting States:
CH DE FR GB IT LI NL SE

(71) Applicant: **MITSUBISHI CHEMICAL INDUSTRIES LIMITED**
5-2, Marunouchi 2-chome Chiyoda-ku
Tokyo 100 (JP)

Shikata, Toshio
1-172 Motokitakata
Ichikawa-shi Chiba-ken (JP)

Ono, Yasushi
2-13-4, Honcho
Shiki-shi Saitama-Ken (JP)

Shimizu, Yohko
3-17-23 Nishiwaseda Shinjuku-ku
Tokyo (JP)

(72) Inventor: **Shikata, Toshio**
1-172 Motokitakata
Ichikawa-shi Chiba-ken (JP)

Ono, Yasushi
2-13-4 Honcho
Shiki-shi Saitama-ken (JP)

Shimizu, Yohko
3-17-23 Nishiwaseda
Shinjuku-ku Tokyo (JP)

Yamada, Ei
8-18-14 Chiyogaoka Asao-ku
Kawasaki-shi Kanagawa-ken (JP)

Honda, Yoshikazu
202 Imai-Koopo 2-3-16 Nishimikado
Kamakura-shi Kanagawa-ken (JP)

Kondo, Jun
7-406 Popuragaoka-Koopu 2-11-1 Naruse
Machida-shi Tokyo (JP)

Maeda, Toshirou
203 Seiwa-Manshon 4-18-24 Soshigaya
Setagaya-ku Tokyo (JP)

(74) Representative: **Peaucelle, Chantal et al**
S.C. Ernest Gutmann - Yves Plasseraud 67, boulevard Haussmann
F-75008 Paris (FR)

(54) Non-A, non-B, hepatitis antigen.

(57) The present invention relates to purified non-A, non-B hepatitis antigens, which have a molecular weight of 44,000 ± 2,000 and a partial amino acid sequence represented by any of the formulae:

(I) Tyr-Asn-Ser-Pro-Thr-Asn-Phe-Gln-Ile-Asp-Gly-Arg-Asn-Arg-Lys;

(II) Val-Ile-Met-Asp-Leu-Lys;

(III) Ser-Ser-Phe-Phe-Asn-Ser-Val-Arg-Ser-Val-Phe-Gln-Gly-His-Val.

EP 0 263 761 A2

**Description**

NON-A,NON-B HEPATITIS ANTIGEN

BACKGROUND OF THE INVENTION

Field of the Invention

The present invention relates to the purified non-A, non-B hepatitis antigen.

Description of the Prior Art

Among viral hepatitis, pathogenic viruses of hepatitis A and B have been found and, therefore, an immunological diagnosis of the diseases has now been possible.

However, pathogenic viruses of another type of hepatitis different from the type A and B, which is called non-A non-B type and said to account for over 90% of post-transfusion hepatitis, have not yet been identified. One fact which has already been established is that a chimpanzee could be infected with human non-A,non-B hepatitis. Many studies for searching an antigen/antibody system related to non-A non-B hepatitis have been done using mainly sera from patients; nevertheless, no effective system has been found.

The present inventors made great efforts to establish such an antigen/antibody system related to non-A,non-B hepatitis and for the first time found an antigen specific to non-A non-B hepatitis, refer to Japanese Patent. Application Laying-open (KOKAI) No. 176856/86. As a result of further studies, the present inventors have now found a purified antigen directed to non-A,non-B hepatitis, said antigen being able to be advantageously utilized as a means for diagnozing non-A,non-B hepatitis which cannot exactly be diagnozed by conventional methods, and thus attained the present invention.

SUMMARY OF THE INVENTION

Accordingly, the principal object of the invention is to provide a purified non-A, non-B hepatitis antigen.

Thus, the invention comprises a purified non-A, non-B hepatitis antigen, which has a molecular weight of 44,000 ± 2,000 and a partial amino acid sequence represented by any of the following formulae (I) to (III):

(I)　Tyr-Asn-Ser-Pro-Thr-Asn-Phe-Gln-Ile-Asp-Gly-Arg-Asn-Arg-Lys;

(II) Val-Ile-Met-Asp-Leu-Lys;

(III) Ser-Ser-Phe-Phe-Asn-Ser-Val-Arg-Ser-Val-Phe-Gln-Gly-His-Val.

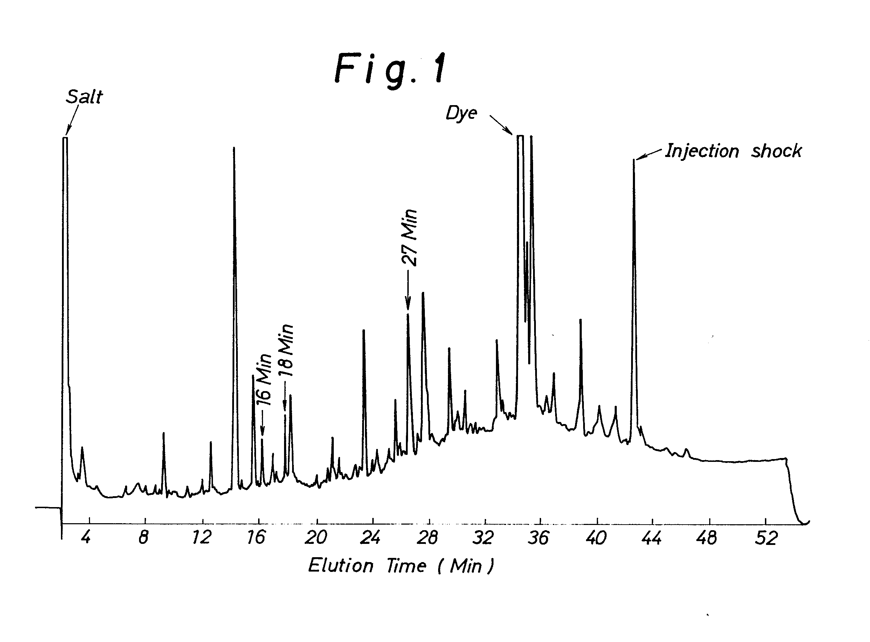

BRIEF DESCRIPTION OF THE DRAWING

The sole figure attached herewith shows the elution pattern on the reverse phase high performance liquid chromatography of the lysyl endopeptidase digests of the present purified non-A, non-B hepatitis antigen obtained in Example described hereinbelow. The abscissa corresponds to the elution time (minute).

DESCRIPTION OF THE INVENTION

The present invention will hereinafter be described in more detail.

The antigens of the invention may be prepared by conventional methods one of the methods will be described hereinbelow.

It comprises homogenizing a liver specimen with non-A, non-B hepatitis, centrifuging the homogenized materials, subjecting the supernatant to sucrose cushion ultra centrifugation, subjecting the resulting precipitate to sucrose density gradient ultra centrifugation, ultra centrifuging a desired fraction, electrophoresing the precipitate on SDS-polyacrylamide gel, and extracting a protein from the gel corresponding to a molecular weight of 44,000 ± 2.000.

Antigen materials may be liver specimens derived from a human or chimpanzee individual with non-A, non-B hepatitis, which herein includes individuals with so-called type D hepatitis.

In a preferred embodiment, such a liver specimen is homogenized and centrifuged at about 10,000 × g for a period of from about 30 minutes to one hour. The resultant supernatant is placed on a buffer containing 15-25% (W/W) sucrose in a centrifugal tube and centrifuged at about 100.000 × g for about 3 to 5 hours to purify.

The pellet is dissolved in a small amount of a buffer and subjected to sucrose density gradient centrifugation to further purify. Preferably, the centrifugation may effected in 10-60% (W/W) sucrose density gradient at 70,000-80,000 × g for about 50 to 60 hours.

After the centrifugation, a desired fraction, which may herein correspond to a sucrose density in the range of from about 1.20 to 1.25, is collected. The fraction is further centrifuged at 100,000 × g for about 3 to 5 hours.

The pellet is dissolved in a small amount of a buffer and subjected to 0.1% SDS (sodium dodecyl sulfate solution)/7-15% polyacrylamide gel electrophoresis at a constant current of 5 to 30 mA for a period of from 45 minutes to 6 hours. The location of a desired protein on the gel is determined by the known Western blotting method using a mouse monoclonal antibody obtained in Reference Example 1 described hereinbelow. In the present invention, it has been found that the location of the desired protein on the gel corresponds to a molecular weight of 44,000 ± 2,000.

The gel corresponding to a molecular weight of 44,000 ± 2,000 is cut and subjected to conventional electrophoresis according to usual procedures to obtain the desired protein attached to SDS (sodium dodecyl sulfate). After removing SDS in a conventional manner, the desired protein, i.e., non-A,non-B hepatitis antigen, can be obtained with a purity of 100%.

In the above described purification process, the desired antigens are assayed by RIA methods as described below.

(A) Radioimmunoassay (RIA)-I:

(i) Iodination of antibody
1. One ml of 0.2 M sodium borate buffer, pH 9.0, containing 10% BSA (bovine serum albumin) is applied to "Sephadex" G-25 column, 0.7 × 20 cm, to block the support resin with albumin.
2. $Na^{125}I$ (500 μCi) is put into a polystyrene tube (75 × 12 mm).
3. 50 μl of human IgM monoclonal antibody, which may be obtained in Japanese Patent Application Laying-open (KOKAI) No.56196/86 (50 μg in 0.2 M sodium borate buffer, pH 9.0), is added to the tube and thoroughly stirred.
4. 50 μl of chloramine T solution (1 mg/ml in 0.2 M sodium borate buffer, pH 9.0) is added and thoroughly stirred to react at room temperature for one minute.
5. 50 μl of sodium metabisulfite (2.5 mg/ml in 0.2 M sodium borate buffer, pH 9.0) is added and thoroughly stirred.
6. 300 μl of potassium iodide solution (1 mg/ml in sodium borate buffer, pH 9.0, containing 0.1% BSA) is added and thoroughly stirred.
7. IgM fraction is fractionized on "Sephadex" G-25 column.

(ii) Radioimmunoassay
1. 50 μl of IgM antibody (100 μg/ml) solubilized in 0.2 M sodium borate buffer, pH 9.0, is adsorbed on "Falcon 3912" 96-well microtiter plate at 4°C over night.
2. The antibody solution is removed, and the plate is washed with 0.01 M phosphate buffer solution (PBS), pH 7.2, to block the plate with 5% BSA-PBS.
3. A standard antigen or test antigen fraction is diluted with 0.25% gelatin-PBS containing 0.05% NP-40 and 0.02% $NaN_3$, and 50 μl of the diluted antigen solution is distributed in duplicate to react at room temperature for 2 to 16 hours.
4. The plate is washed with PBS-"Tween" 20 (0.1%).
5. $^{125}I$-Labelled antibody is diluted with gelatin-PBS, and 50 μl (5 × $10^4$ cpm) of the diluted antibody solution is added to each well and reacted at room temperature for 2 to 16 hours.
6. The plate is washed with PBS-"Tween" 20.
7. Each well is cut and its radioactivity is measured by a gamma counter.

(B) Radioimmunoassay (RIA)-II:

(i) Iodination of antibody
1. One ml of 0.01 M phosphate buffered saline (PBS), pH 7.2, containing 10% BSA (bovine serum albumin) is applied to "Sephadex" G-25 column, 0.7 × 20 cm, to block the support resin with albumin.
2. $Na^{125}I$ (500 μCi) is put into polystyrene tubes (75 × 12 mm).

3. 10 μg of mouse IgG diluted with 50 μl of PBS is added to the tube and thoroughly stirred.
4. 50 μl of chloramine T solution (Sigma, 1 mg/ml in PBS) is added and thoroughly stirred to react at room temperature for one minute.
5. 50 μl of sodium metabisulfite (2.5 mg/ml in PBS) is added and thoroughly stirred.
6. 250 μl of potassium iodide solution (1 mg/ml in PBS) and 250 μl of BSA (1 mg/ml in PBS) are added and thoroughly stirred.
7. IgG fraction is fractionized on "Sephadex" G-25 column.

(ii) Radioimmunoassay
1. 50 μl of IgG antibody (100 μg/ml) solubilized in 0.01 M phosphate buffered saline (PBS), pH 7.2, is adsorbed on "Falcon 3912" 96-well microtiter plate at 4°C over night.
2. The antibody solution is removed, and the plate is blocked with 5% BSA-PBS.
3. A standard antigen or test antigen fraction is diluted with 0.25% gelatin-PBS containing 0.05% NP-40 and 0.02% $NaN_3$ and 50 μl of the diluted antigen solution is distributed in duplicate to react at room temperature for 2 hours.
4. The plate is washed with PBS-"Tween" 20 (0.1%).
5. $^{125}I$-Labelled antibody is diluted with gelatin-PBS, and 50 μl (5 × $10^4$ cpm) of the diluted antibody solution is added to each well and reacted at room temperature for 2 hours.
6. The plate is washed with PBS-"Tween" 20.
7. Each well is cut and its radioactivity is measured by a gamma counter.

The thus prepared antigen according to the invention specifically reacts with mouse specific antibody as shown in Reference Example 2 described hereinbelow. It has a molecular weight in the range of from 42,000 to 46,000 (44,000 ± 2,000) and an isoelectric point (pH) in the range of from 6.8 ± 0.5 to 7.2 ± 0.5.

The elution pattern on the reversed phase high performance liquid chromatography (under the conditions shown in Example described hereinbelow) of materials obtained by digesting the antigen with lysyl endopeptidase is shown in the sole figure attached herewith. The fractions eluted at about 18, 16 and 27 minutes in the pattern have a partial amino acid sequence represented by one of the following formulae, respectively:
(I)    Tyr-Asn-Ser-Pro-Thr-Asn-Phe-Gln-Ile-Asp-Gly-Arg-Asn-Arg-Lys;
(II) Val-Ile-Met-Asp-Leu-Lys;
(III)    Ser-Ser-Phe-Phe-Asn-Ser-Val-Arg-Ser-Val-Phe-Gln-Gly-His-Val.

The antigens according to the invention have a purity of 100% and, accordingly, are very useful as diagnostic means for non-A,non-B hepatitis.

DESCRIPTION OF THE PREFERRED EMBODIMENTS
The following examples will fully illustrate the present invention but they are not intended to limit the scope of the invention defined in the attached claims.

## REFERENCE EXAMPLE 1:

### Preparation of Mouse Specific Antibody against Non-A,Non-B Hepatitis Antigens

#### (1) Culture Medium:

After adding 100 µg/ml lilacillin, 100 µg/ml streptomycin, 2 mM glutamine and 1.6 g/1 sodium bicarbonate to RPMI 1640 medium, carbon dioxide was blown thereinto to adjust the pH to 7.0-7.4, and fetal calf serum (FCS) was added to 10%.

#### (2) Myeloma Cell Line:

The established cell line, P3-X63-Ag8-U1 (P3U1), derived from Balb/c mouse bone marrow cell MO-PC-21, was used. The cell line was 8-azaguanine resistant and globulin non-secretor.

#### (3) Antigen:

Antigens used in immunization and antibody assay were obtained according to the method of Example.

#### (4) Splenocyte:

The purified non-A,non-B hepatitis related antigen (10 mg/ml) was mixed with an equal amount of Freund's complete adjuvant. The resulting emulsion was intraperitoneally injected to Balb/c mice in an amount of 0.25 mg (0.5 ml) per animal for immunization. After 5 weeks, the mice were boosted either by intraperitoneal injection of 0.5 mg antigen (0.5 ml) per animal or by tail-intravenous injection of 0.3 mg antigen (0.3 ml) per animal. Four days after the booster the splenocytes were used in the following cell fusion.

#### (5) Cell Fusion:

Both the myeloma cells and splenocytes were three times washed with Hanks' solution and suspended in RPMI 1640 containing 10% FCS to determine the number of cells. From 7.5 to 10 × 10⁶ splenocytes were cultured with 1 × 10⁶ P3U1 cells for 2-3 hours. The cells were then washed by centrifugation in Minimum Essential Medium (MEM) to remove FCS. The cells were collected in a centrifuge tube.

After removing thoroughly the supernatant, the cell pellet was disentangled. PEG solution (MEM containing 42.5% PEG-1000) which had been warmed to 37°C was added in an amount of 0.5 ml. After treatment at room temperature for one minute, ten portions of MEM (37°C) were added each in an amount of one ml at intervals of 30 seconds, during which period the centrifuge tube was slowly rotated.

After the cell fusion, the cells were washed by centrifugation. RPMI 1640 containing 10% FCS was added so that the number of P3U1 cells was in the range of 5 to 10 × 10⁵ per ml. The cell solution was distributed into a microtiter plate in an amount of 0.2 ml per well. After 24 hour culture, a half volume of the supernatant was discarded and, instead, HAT (hypoxanthin, aminopterine and thymidine) medium was added to each well. This procedure was repeated for 2 weeks at intervals of 48 hours.

The myeloma and splenocyte cannot grow in HAT medium. Therefore, cells growing in the medium may be considered to be hybridomas. After 10 to 14 days, only the cultures of wells in which any hybridoma was observed to grow were assayed for their antibody activity.

#### (6) Screening of Antibody Activity:

Antibody activities were screened according to the following radioimmunoassay methods.

##### Radioimmunoassay-I:

1. 50 µl of IgM antibody (100 µg/ml; obtained by EB virus-transformation method described in Japanese Patent Application Laying-open (KOKAI) No. 56196/86) solubilized in 0.2 M sodium borate buffer, pH 9.0, is adsorbed on "Falcon 3912" 96-well microtiter plate at 4°C over a whole day and night.

2. The antibody solution is removed, and the plate is washed with 0.01 M phosphate buffer solution (PBS), pH 7.2, and blocked with 5% BSA-PBS.

3. A standard antigen sample is diluted with 0.25% gelatin-PBS containing 0.05% NP-40 and 0.02% NaN₃, and 50 µl of the diluted antigen solution is distributed to each well to react at room temperature for 2 to 16 hours.

4. The plate is washed with PBS-"Tween-20" (0.1%).

5. A culture sample of a well containing any hybridoma is distributed in an amount of 50 µl per well and the reaction is allowed to proceed at room temperature for 2 to 16 hours.

6. The plate is washed with PBS-"Tween-20".

7. ¹²⁵I-Labelled anti-mouse immunoglobulin antibody is diluted with gelatin-PBS, and 50 µl (5 × 10⁴ cpm) of the diluted antibody solution is added to each well and reacted at room temperature for 2 to 16 hours.

8. The plate is washed with PBS-"Tween-20".

9. Each well is cut and its radioactivity is measured by a gamma counter.

##### Radioimmunoassay-II:

1. 50 µl of IgG anti-mouse immunoglobulin antibody (10 µg/ml) solubilized in PBS is adsorbed on "Falcon 3912" 96-well microtiter plate at 4°C over a whole day and night.

2. The antibody solution is removed, and the plate is washed with 0.01 M PBS and blocked with 5% BSA-PBS.

3. A culture sample of a well containing any hybridoma is distributed in an amount of 50 µl per well and the reaction is allowed to proceed at room temperature for 2 to 16 hours.

4. The plate is washed with PBS-"Tween-20".

5. A standard antigen sample is diluted with gelatin-PBS and 50 µl of the diluted antigen solution is distributed to each well to react at room temperature for 2 to 16 hours.

6. The plate is washed with PBS-"Tween-20".

7. ¹²⁵I-Labelled IgM antibody obtained by the EB virus-transformation method (Japanese Patent Application Laying-open (KOKAI) No. 56196/86) is diluted with gelatin-PBS, and 50 µl ($5 \times 10^4$ cpm) of the diluted antibody solution is added to each well and reacted at room temperature for 2 to 16 hours.

8. The plate is washed with PBS-"Tween-20".

9. Each well is cut and its radioactivity is measured by a gamma counter.

The supernatant of the culture containing a hybridoma obtained according to the method described above was diluted successively to $2^n$ times in a microtiter plate, and the maximum dilution estimated to be positive in the radioimmunoassay was employed as a titer of the antibody secreted by the hybridoma.

### (7) Cloning:

Cloning was effected by the limiting dilution method. Mouse splenocytes were suspended in RPMI 1640 medium containing 15% FCS to a cell density of $10^7$ per ml and used as a feeder layer. The hybridoma cell was diluted to 1, 0.2 or 0.1 cell per well and 0.1 ml of the diluted hybridoma cell culture was inoculated in each well of a 96 well microtiter plate. After one week, wells containing only one colony were searched by an inverted microscope and assayed by the above described RIA.

These cloning procedures were further repeated several times. Clones which stably produced an antibody and actively grew were selected.

### (8) Collection and Purification of Antibody:

The clones were allowed to grow in a serum-free NYSF-404 medium containing 0.5% bovine serum albumin (BSA) and the culture supernatants were collected.

The supernatant was subjected to salting-out with ammonium sulfate to concentrate. Affinity chromatography on "Protein A Sepharose"-CL-4B ("MAPS buffer", Bio-Rad) was effected to obtain a purified antibody.

The mouse purified antibody showed a precipitation curve corresponding to an IgG antibody in Ouchterlony's method and immunoelectrophoresis. Two bands corresponding to H and L chains of IgG were observed in SDS-polyacrylamide gel electrophoresis under reducing conditions.

### REFERENCE EXAMPLE 2: Reactivities of the Antibody

### (1) Reactivity with Liver Specimens of Non-A,Non-B Hepatitis:

The antibody obtained in Reference Example 1 was assayed for the presence of the reactivity with liver specimens with non-A,non-B hepatitis by immunofluorescence method.

Thus, liver specimens sliced by a cryostat were treated with acetone at room temperature for 10 minutes. After drying, the above described non-A,non-B hepatitis-related antibody as a primary antibody was placed on the slice and allowed to react at 37°C for 60 minutes in a wet box. The antibody solution had previously been diluted to an appropriate concentration.

After washing out the primary antibody with PBS, the specimen was washed with PBS under agitation. Then, FITC-labelled anti-mouse immunoglobulin antibody as a secondary antibody was reacted with the specimen at 37°C for 60 minutes in a similar manner as described above. After washing out the labelled antibody with PBS, the specimen was washed in PBS under agitation and then air dried. A sealing agent, glycerin: 0.5 M $Na_2CO_3$-$NaHCO_3$ buffer (9:1), was dropwise added and a cover glass was mounted thereon. The specimen was observed by a fluorescence microscope with a mercury lamp as a light source.

The results showed that the antibody reacted with the antigen appearing in the liver with non-A,non-B hepatitis.

### (2) Reactivities with Liver Specimens of Other Hepatitis and Normal Liver Specimens:

In the same manner as described in (1) above, the antibody obtained in Reference Example 1 was assayed for the reactivities with liver specimens other than those of non-A, non-B hepatitis by the immunofluorescence method.

The results showed that the antibody did not react with liver specimens of hepatitis A or B, nor with those of non-viral hepatitis, nor with normal liver specimens.

### EXAMPLE: Isolation and Purification of Antigens
### Isolation and Purification of Antigens Appearing in Chimpanzee Hepatocytes with Non-A,Non-B Hepatitis

(1) A hepatocyte mass, 10 g, of a chimpanzee suffering from non-A,non-B hepatitis was minced into small pieces and suspended in cold Tris buffer (50 mM Tris-HCl buffer containing 0.13 M NaCl and 5 mM EDTA, pH 7.4) so that the total volume was 100 ml. The hepatocyte suspension was then homogenized in a ultra high speed universal homogenizer, HISCOTRON, K.K. NICHI-ON IRIKA KIKAI SEISAKUSHO, Japan, the vessel outer wall of which was adapted to be well cooled at 0°C, and centrifuged at $10,000 \times g$, 4°C for one hour. Thus, 90 ml of the supernatant was collected.

(2) The resultant samples were layered onto 5 ml of 20% (W/W) sucrose in Tris buffer in centrifugal tubes, each 30 ml of the sample per tube, and ultracentrifuged at 4°C, $100,000 \times g$ for 4 hours. The thus separated supernatant and precipitate which was suspended and solubilized in 2.5 ml of cold Tris buffer were each assayed for the antigen activity by the aforementioned RIA. (Identification of a desired protein in the following purification process was effected by the RIA.) The results showed that 95% or more antigen activity was recovered in the precipitate.

(3) The thus obtained antigen fraction (2.5 ml per tube) was layered onto 32.5 ml of Tris buffer containing 10-60% (W/W) sucrose. Sucrose density gradient centrifugation was effected at $77,000 \times g$, 4°C for 60 hours. Each fraction (2.5 ml per tube) was assayed for the antigen activity by RIA. The antigen was recovered in fractions having a sucrose density of 1.20 to 1.25.

(4) The thus obtained antigen fractions (10 ml) were mixed with 25 ml of cold Tris buffer. Ultracentrifugation was effected at 100,000 × g, 4°C for 4 hours to divide the mixture into a precipitate and a supernatant.

(5) The precipitate containing about 2 mg of protein was dissolved in a small amount of a sample buffer for SDS-polyacrylamide gel electrophoresis, which was 50 mM Tris-HCl buffer, pH 6.8, containing 2% SDS, 5% β-mercaptoethano1,10% glycerin and 0.005% Bromophenol Blue. The total volume of the resulting solution was adjusted to 2 ml with the same sample buffer. The resultant solution was heated at 100°C for 3 minutes.

(6) The thus obtained sample (10 μl) containing 10 μg protein was added to 0.1% SDS-7.5% polyacrylamide gel having a size of 70 × 85 × 1 mm. Simultaneously, Pharmacia low molecular weight marker protein "LMW KitE" was also added as marker proteins. By using Tris buffer (25 mM Tris, pH 8.3, 192 mM glycine, 0.1% SDS) as an electrode solution, electrophoresis was effected at a constant current of 30 mA for 45 minutes. According to conventional methods, said gel was put onto a nitrocellulose filter (S&S "BA-83", pore size of 0.2 μm), and a constant voltage of 24.5 V was applied for 2 hours between the gel as a cathode and the nitrocellulose filter as an anode.

(7) Either marker protein portions or sample portions were each cut from the thus transferred nitrocellulose filter. The marker protein portions were stained with 0.1% (w/v) Amido Black 10B, while the sample portions were immersed into 0.01 M phosphate buffer solution, pH 7.2, containing 5% (w/v) bovine serum albumin at room temperature for 2 hours.

(8) 200 μl of mouse monoclonal IgG antibody (20 μg/ml), prepared in Reference Example 1, was placed onto the filter of the sample portions, and allowed to stand at room temperature for 2 hours. The filter was three times washed with PBS containing 0.1% (v/v) of Sigma Tween 20, each time for 20 minutes.

(9) Onto the filter of the sample portions, 200 μl of peroxidase-labelled anti-mouse IgG antibody (Cappel), diluted to 1/500 times, was placed, and the reaction was allowed to proceed at 37°C for one hour. The filter was three times washed with PBS containing 0.1% (v/v) Tween 20, each time for 20 minutes.

(10) The sample filter was immersed into 100 ml of a peroxidase color developer solution (60 mg 4-chloro-1-naphthol, 20 ml methanol, 80 ml PBS, 20 μl aqueous hydrogen peroxide solution).

(11) The colored filter was washed with distilled water and compared to the filter of said marker protein portions.

The results showed that the mouse monoclonal antibody recognized a protein having a molecular weight of about 44,000.

(12) The remainder of the sample prepared in (5) above was subjected to SDS-polyacrylamide gel electrophoresis in the same manner as in (6) above. After staining, the gel corresponding to proteins having a molecular weight of about 44,000 was cut and sliced into pieces of about 1 mm square. The pieces were added to a cathodic well of "Maxyield GP", which was an apparatus for electrophoresis manufactured by ATTO.

A solution for protein extraction, which consisted of an aqueous solution of 0.4 M triethylamine containing 2% SDS and 0.1% dithiothreitol, was added to the cathodic well so that the total volume was 14 ml, and allowed to stand at room temperature for 4 hours.

An aqueous solution of 50 mM triethylamine containing 0.1% SDS as an electrode solution was added to the well in an amount of 30 ml. The well was fitted into an electrophoretic tank filled with the same aqueous solution. The electrophoresis was effected at a constant voltage of 100 V at 4°C for 24 hours.

The electrode solution in the electrophoretic tank was replaced with a fresh electrode solution (an aqueous solution of 10 mM triethylamine containing 0.02% SDS). The electrophoresis was further effected at a constant voltage of 100 V at 4°C for 24 hours.

Proteins and dye stuffs were moved toward the anode. After the phoresis, about 2 ml of the colored protein-containing gel fraction was recovered from the anodic well and lyophilized to obtain the protein.

(13) The protein was dissolved in 500 μl of anhydrous acetone containing 1% (v/v) triethylamine. Centrifugation was effected at 15,000 × g, 4°C for 5 minutes. The supernatant was discarded. These procedures were repeated three times. SDS and dyes were removed out. Thus, about 84 μg of the desired protein having a purity of 100% was obtained.

(14) The protein (about 10 μg) was suspended in 50 μl of a reagent solution for isoelectric focusing (8.5 M urea, 4% Nonidet P-40, Sigma, 5% 2-mercaptoethanol, 2% Ampholine, LKB, pH 3.5-9.5).

(15) Said sample (50 μl) was added to 4% polyacrylamide gel, having a degree of crosslinking of 4% and containing 8.5 M urea, 2% Ampholine and 4% NP-40, which had been charged into a glass tube of 3.5 mm in diameter and 70 mm in height. 50 μl of a protective solution (5 M urea, 2% Ampholine, 2% NP-40) was placed onto the sample. 0.02 N NaOH was added to an upper electrode tank, while 0.01 M $H_3PO_4$ to a lower electrode tank. Electrophoresis was effected at 10°C and at a constant voltage of 300 V for 16 hours with the upper being the cathode and the lower the anode, and then at a constant voltage of 400 V for one hour.

(16) The gel was removed out from the glass tube and immersed into 20 ml of the sample buffer for SDS-polyacrylamide gel electrophoresis at room temperature for 30 minutes.

(17) The thus treated gel was fixed on 0.1% SDS-7.5% polyacrylamide gel (70 × 85 × 2 mm) by means of 1% (w/v) agarose, and electrophoresed at a constant current of 30 mA for 90 minutes.

After the electrophoresis, the gel was stained with Coomassie Briliant Blue. Spots of protein were observed in portions corresponding to pH 6.8-7.2.

It was found that the protein spots reacted with said mouse monoclonal antibody by Western blotting method using the gel obtained by electrophoresis in the same manner as above except that the electrophoresed gel was not stained.

(18) About 30 μg of the protein obtained in (13) above was dissolved in 100 μl of Tris buffer (50 mM Tris-HCl buffer, pH 9.0, containing 5 M urea), and digested at 37°C for 6 hours with lysyl endopeptidase in an amount of 1/400 times by mole of the amount in mole of the protein.

(19) The digests were separated on reverse phase high performance liquid chromatography using "Cosmosil" 5C$_8$-300 column, NAKARAI KAGAKU, Japan, of 0.46 × 15 cm. The elution was effected by applying an aqueous solution of 0.1% trifluoroacetic acid containing linear density gradient of 0-70% acetonitrile at a flow rate of one ml per minute over 50 minutes. Peptides were detected by absorption at 215 nm. The results are shown in the sole figure attached herewith. As seen from the figure, about 26 fractions were obtained.

(20) Among these fractions, those eluted at about 16, 18 and 27 minutes were dried under vacuum, dissolved in 60 μl of 60% trifluoroacetic acid, added to a glass filter treated with polybrene, and subjected to Edman degradation in a sequencer manufactured by Applied Biosystems, 470A type.

(21) PTH-amino acids were eluted from "TSK gel ODS-80 T$_M$", manufactured by TOYO SODA MANUFACTURING CO., LTD., 0.46 × 15 cm, by applying an acetic acid buffer (10 mM acetic acid, pH 4.5, containing 0.008% SDS and 38% acetonitrile) as a solvent at a flow rate of one ml per minute, and detected by absorbance at 254 nm.

(22) It was evident from the results that these peptides had an amino acid sequence represented by the following formula:

(I) Tyr-Asn-Ser-Pro-Thr-Asn-Phe-Gln-Ile-Asp-Gly-Arg-Asn-Arg-Lys for 18 min eluted fraction;

(II) Val-Ile-Met-Asp-Leu-Lys for 16 min eluted fraction;

(III) Ser-Ser-Phe-Phe-Asn-Ser-Val-Arg-Ser-Val-Phe-Gln-Gly-His-Val for 27 min eluted fraction.

## Claims

1. A purified non-A, non-B hepatitis antigen, which has a molecular weight of 44,000 ± 2,000 and a partial amino acid sequence representend by any of the formulae :

(I) Tyr-Asn-Ser-Pro-Thr-Asn-Phe-Gln-Ile-Asp-Gly-Arg-Asn-Arg-Lys;

(II) Val-Ile-Met-Asp-Leu-Lys;

(III) Ser-Ser-Phe-Phe-Asn-Ser-Val-Arg-Ser-Val-Phe-Gln-Gly-His-Val.

2. Antigen according to claim 1, characterized by the fact it reacts with mouse specific antibody, it has a molecular weight in the range of from 42,000 to 46,000 (44,000 ± 2,000) and an isoelectric point (pH) in the range of from 6.8 ± 0.5 to 7.2 ± 0.5.

3. Antigen according to claim 1 or 2, characterized by the fact that the materials obtained by digesting the antigen with lysyl endopeptidase have an elution pattern on the reversed phase high performance liquid chromatography as shown on the sole figure.

4. A process for preparing a purified non-A, non-B hepatitis antigen, which has a molecular weight of 44,000 2,000 and a partial amino acid sequence represented by any of the formulae :

(I) Tyr-Asn-Ser-Pro-Thr-Asn-Phe-Gln-Ile-Asp-Gly-Arg-Asn-Arg-Lys;

(II) Val-Ile-Met-Asp-Leu-Lys;

(III) Ser-Ser-Phe-Phe-Asn-Ser-Val-Arg-Ser-Val-Phe-Gln-Gly-His-Val.

said process comprising homogenizing a liver specimen from a human or chimpanzee individual with non-A, non-B hepatitis, centrifuging the homogenized materials, subjecting the supernatant to sucrose cushion ultra centrifugation, subjecting the resulting precipitate to sucrose density gradient ultra centrifugation, ultra centrifuging a desired fraction, electrophoresing the precipitate on SDS-polyacrylamide gel, and extracting a protein from the gel corresponding to a molecular weight of 44,000 ± 2,000.

5. The process of claim 4 comprising homogenizing a liver specimen from a human or chimpanzee individual with non-A, non-B hepatitis, at about 10.000 g for a period of from about 30 minutes to one hour, placing the resultant supernatant on a buffer containing 15-25% (W/W) sucrose in a centrifugal tube and centrifuging at about 100,000 × g for about 3 to 5 hours to purify, dissolving the pellet in a small amount of a buffer and subjecting to sucrose density gradient centrifugation to further purify, collecting a fraction corresponding to a sucrose density in the range of from about 1.20 to 1.25, further centrifuging said fraction at 100,000 × g for about 3 to 5 hours, dissolving the pellet in a small amount of a buffer and subjecting to 0.1% SDS (sodium dodecyl sulfate solution)/7-15% polyacrylamide gel electrophoresis at a constant current of 5 to 30 mA for a period of from 45 minutes to 6 hours and extracting the desired protein from the gel.

6. The process of claim 5. wherein the centrifugation is carried out in 10-60% (W/W) sucrose density gradient at 70,000-80,000 × g for about 50 to 60 hours.

7. A method for the in vitro diagnostic for non-A, non-B hepatitis, comprising the use of an antigen according to anyone of claims 1 to 3.

0263761

*Fig. 1*

Injection shock

Dye

Salt

27 Min

18 Min

16 Min

Elution Time ( Min )

4  8  12  16  20  24  28  32  36  40  44  48  52